Europäisches Patentamt

European Patent Office

Office européen des brevets

(11) Publication number: **0 199 548**
A2

(12) # EUROPEAN PATENT APPLICATION

(21) Application number: **86302878.3**

(51) Int. Cl.⁴: **C 12 P 19/02**

(22) Date of filing: **17.04.86**

(30) Priority: **22.04.85 JP 86747/85**

(43) Date of publication of application:
**29.10.86 Bulletin 86/44**

(84) Designated Contracting States:
**AT BE CH DE FR GB IT LI LU NL SE**

(71) Applicant: **Takeda Chemical Industries, Ltd.**
**27, Doshomachi 2-chome Higashi-ku**
**Osaka-shi Osaka, 541(JP)**

(72) Inventor: **Kintaka, Kazuhiko**
**7-2, Asae 1-chome**
**Hikari Yamaguchi 743(JP)**

(72) Inventor: **Yoshinaga, Hiroyuki**
**10-4, Ebisu-machi**
**Hikari Yamaguchi 743(JP)**

(72) Inventor: **Yamaguchi, Takamasa**
**D-75-206, Tsukumodai 5-chome**
**Suita Osaka 565(JP)**

(74) Representative: **Lewin, John Harvey et al,**
**Elkington and Fife High Holborn House 52/54 High**
**Holborn**
**London WC1V 6SH(GB)**

(54) **Method for producing L-sorbose.**

(57) In a method for producing L-sorbose by microbial oxidation of D-sorbitol, a competent microorganism is cultivated in a medium in which the concentration of dissolved oxygen is controlled in the range of about 1 to about 4 ppm throughout the growth phase of said microorganism. Thus, L-sorbose can be produced in much greater yield, compared with known methods.

EP 0 199 548 A2

- 1 -

## Method for Producing L-sorbose

The present invention relates to a microbial method for producing L-sorbose.

L-Sorbose is of value as a starting material for synthesis of vitamin C. Heretofore, L-sorbose has been produced by oxidizing D-sorbitol with a microorganism such as one belonging to the genus Gluconobacter.

In the conventional fermentative method for production of L-sorbose, the yield of conversion from D-sorbitol to L-sorbose is about 93 percent at the maximum and, in addition, 5-ketofluctose, D-fructose or/and 2-ketogluconic acid are by-produced in fairly large amounts. For the purpose of reducing the cost of materials in the production of vitamin C, it has been required to increase the conversion yield from D-sorbitol to L-sorbose and inhibit formation of the above-mentioned by-products as much as possible.

In view of the above situation, the present inventors made an intensive research to develop an efficient method for producing L-sorbose and have completed the present invention.

The present invention is directed to an improved method for producing L-sorbose by microbial oxidation of D-sorbitol, which comprises cultivating a competent microorganism in a medium in which the concentration of dissolved oxygen is controlled in the range of about 1 to about 4 ppm throughout the growth phase of said microorganism.

The production method according to the present invention can be practiced by using any microorganism that is able to oxidize D-sorbitol to L-sorbose. Examples of such microorganism include various bacteria of the genus Gluconobacter, such as Gluconobacter suboxydans and Gluconobacter oxydans. The following is a partial listing of competent strains of these species. Gluconobacter suboxydans IFO 3254, IFO 3257, IFO 12528, IFO 3255, IFO 3256, IFO 3258 and IFO 3291 and Gluconobacter oxydans IFO 3189.

The strains exemplified above have been listed on List of Cultures, 1984, Seventh Edition issued by Institute for Fermentation, Osaka (IFO) and deposited in the same institute.

The term "growth phase of a competent microorganism" is used herein to denote the cultivation time including the induction or lag phase to the logarithmic growth phase of the particular microorganism cultivated. This growth phase varies with different microorganisms and cultivating conditions employed but it can be easily found by constructing a growth curve in accordance with the conventional procedure. Generally, it is the cultivation time till complete consumption of nitrogen sources available in the medium and corresponds, in many instances, to a time period of about 9 hours after initiation of cultivation.

In the present invention, the concentration of dissolved oxygen in the medium is controlled at about 1 to 4 ppm throughout the growth phase. During this period, it is advantageous to minimize the variation of dissolved oxygen concentration. Generally, the variation is controlled within the range of ± about 0.5 ppm of a set value chosen from the above-mentioned concentration range. The preferred range of variation is within about 0.3 ppm.

The dissolved oxygen concentration is controlled by monitoring the concentration in the medium on a continuous basis and adjusting the aeration rate, internal pressure, stirring conditions and other cultivation parameters from time to time as fermentation proceeds. The monitoring can be preferably conducted by a method using oxygen electrode as the sensor for dissolved oxygen: The dissolved oxygen concentration, for example, can be controlled at the specified level by adjusting the rate of aeration within the range of about 0.1 to about 1.0 v.v.m. and the internal pressure within the range of 0 to 2.0 $kg/cm^2$ gauge, in suitable combinations thereof in a stepwise manner. The stirring speed may also be adjusted to the same end, if necessary.

After the growth phase, the dissolved oxygen concentration in the medium need not be controlled at the specified level. Generally, the cultivation may be continued at the level of about 0.5 to about 4 ppm under the sparging and stirring conditions obtaining at the end of the growth phase.

The present invention is characterized by controlling the concentration of dissolved oxygen in the medium in the range of about 1 to about 4 ppm throughout the growth phase of the competent microorganism. The other conditions may be employed in the following manner.

As the carbon source in the medium, D-sorbitol may be employed as the main material, and D-glucose, D-fructose, D-mannitol, ethanol, etc. may be added thereto. Generally, cultivation is started at a D-sorbitol concentration of about 10 to 50 w/v %, preferably about 30 to 50 w/v %.

As the nitrogen source, there may be used corn steep liquor, cotton seed meal, yeast extract, dry yeast, fish meal, meat extract, peptone, casamino acids, other nitrogen-containing organic materials, ammonium sulfate, ammonium nitrate, ammonium acetate, ammonium phosphate, ammonium chloride, ammonia water, ammonia gas, and other inorganic nitrogen compounds, amino acids such as sodium glutamate, alanine and the like, urea and other organic nitrogen compounds.

In the present invention, a synthetic medium can be employed more advantageously as a main medium. In this case, as the carbon source in the synthetic medium, there may be used D-sorbitol as the main material, and D-glucose, D-fructose, D-mannitol or ethanol may be added thereto. As the source of nitrogen source, there may be mentioned nitrogen sources such as ammonium sulfate, ammonium nitrate, ammonium acetate, ammonium phosphate or sodium glutamate. Especially, ammonium acetate can be more advantageously used. In addition to the above-mentioned carbon and nitrogen sources, various metals, vitamins, amino acids and nucleic acids required for the growth of microorganisms may optionally be added.

A synthetic medium preferably used in the present invention is exemplified as follows:

| | % (weight/volume) |
|---|---|
| D-Sorbitol | 10 $\sim$ 40% |
| Potassium dihydrogen phosphate | 0.005 $\sim$ 0.1% |
| Magnesium sulfate (heptahydrate) | 0.005 $\sim$ 0.05% |
| Calcium carbonate | 0.005 $\sim$ 0.05% |
| Ammonium acetate | 0.02 $\sim$ 0.05% |
| Sodium glutamate | 0.05 $\sim$ 0.2% |
| Ferrous sulfate (heptahydrate) | 0.00005 $\sim$ 0.0005% |
| Manganese sulfate | 0.0000005 $\sim$ 0.00005% |
| Nicotinamide | 0.0001 $\sim$ 0.01% |
| Calcium pantothenate | 0.00001 $\sim$ 0.001% |
| Paraaminobenzoic acid | 0.000001 $\sim$ 0.0001% |
| Vitamin $B_2$ | 0.00001 $\sim$ 0.001% |

In the present invention, the cultivation conditions other than the dissolved oxygen concentration are not critical but may be the same as those conventionally employed.

Thus, the cultivation temperature is generally about 15°C to 45°C, preferably 25°C to 45°C, and the pH of the medium is generally about 3.0 to 8.0, preferably about 3.5 to 6.5. The cultivation time is generally about 10 to 100 hours, preferably 15 to 40 hours.

After cultivation, L-sorbose is recovered from the culture broth by the conventional method. Thus, for example, culture broth is filtered, decolorized using activated carbon, concentrated under reduced pressure and crystallized with methanol, ethanol, acetone and the like to give L-sorbose.

In accordance with the invention, the yield of L-sorbose in its production by microbial oxidation of D-sorbitol can be increased as compared with the conventional method. Thus, whereas the yield of L-sorbose as attainable in the conventional method is at most about 93% on the D-sorbitol basis, the method according to the invention can increase the yield by at least 2-3% as compared with the conventional method and, moreover, can reduce the formation of byproducts such as D-fructose, 2-ketogluconic acid and 5-keto-fructose. As a result, L-sorbose, which accounts for a very high percentage of the cost of raw materials in the production of vitamin C, can be supplied at a lower cost as compared with the conventional method and consequently the cost of production of vitamin C, which has found wise use in the pharmaceutical and food industries, among others, can be reduced.

Experimental Example 1

Using the same microorganism and medium as described later in Example 1, cultivation was conducted under the same conditions as Example 1 except that the concentration of dissolved oxygen in the medium was varied in the growth phase. The yields of L-sorbose, D-fructose, 2-ketogluconic acid and 5-ketofructose on the D-sorbitol basis as determined after cultivation are shown in Table 1.

- 6 -

Table 1

| No. | Dissolved oxygen concentration (ppm) | L-Sorbose (%) | D-Fructose (%) | 2-Keto-gluconic acid (%) | 5-Keto-fructose (%) |
|---|---|---|---|---|---|
| 1 | 1.5 ± 0.5 | 96.1 | 1.32 | 0.25 | 0.03 |
| 2 | 2.5 ± 0.5 | 95.8 | 1.32 | 0.25 | 0.03 |
| 3 | 3.5 ± 0.5 | 95.9 | 1.33 | 0.20 | 0.04 |
| 4 | 5.5 ± 0.5 | 93.7 | 1.42 | 0.44 | 0.10 |
| 5 | 6.5 ± 0.5 | 93.4 | 1.39 | 0.51 | 0.12 |
| 6 | ≥7 (not controlled) | 93.0 | 1.40 | 0.54 | 0.13 |

In Table 1, L-sorbose, D-fructose, 5-ketofructose and 2-ketogluconic acid were all determined by high performance liquid chromatography. Thus, for L-sorbose, Waters Sugar Pack 1 (Waters Co.) was used as the column, $10^{-4}$ M aqueous solution of EDTA calcium salt as the mobile phase, and a high-sensitivity differential refractometer as the detector. D-Fructose and 5-ketofructose were determined by the arginine color reaction method using an ISA-07/S2504 column (Shimadzu Co., Japan), 3% boric acid (pH 8.0; mobile phase) and a fluorophotometer. 2-Ketogluconic acid was determined by the ultraviolet absorptiometric method (210 nm) using a Shodex Ion Pack C-811 column (Showa Denko Co., Japan) and 0.85% phosphoric acid as the mobile phase.

By performing the cultivation while controlling the dissolved oxygen concentration in medium in the growth phase within the range specified by the present invention (see Run Nos. 1, 2 and 3), the production of byproducts such as 5-ketofructose, D-fructose and 2-ketogluconic acid can be reduced and the yield of the desired product L-sorbose can be increased as compared with the cases where the dissolved oxygen concentration is higher (see Run Nos. 4, 5 and 6), as shown by the results given in Table 1.

### Experiment Example 2

Using the same microorganism and medium as described in Experiment Example 1, cultivation was conducted under the same conditions as Experiment Example 1 except that the concentration of dissolved oxygen in the medium was varied in the growth phase as shown in Table 2.

Table 2

| No. | Dissolved oxygen concentration (ppm) | Yield of L-Sorbose (%) |
|---|---|---|
| 1 | 2 ± 0.3 | 97.0 |
| 2 | 2 ± 0.6 | 95.0 |
| 3 | 2 ± 1.0 | 94.0 |

In case that the variation of dissolved oxygen concentration is controlled within 0.3 ppm, L-sorbose is produced in higher yield as compared with the variations of ±0.6 or ±1.0 ppm, as shown by the results given in Table 2.

### Example 1

Gluconobacter suboxydans IFO 3254 was inoculated into 10 ℓ of a medium containing 20% of D-sorbitol, 0.2% of sodium glutamate, 0.018% of calcium carbonate, 0.003% of nicotinamide, 0.0003% of calcium pantothenate, 0.0001% of vitamin $B_2$, 0.0001% of paraaminobenzoic acid, 0.047% of potassium dihydrogen phosphate, 0.03% of yeast extract, 0.01% of magnesium sulfate, 0.00015% of ferrous sulfate, 0.00001% of manganese sulfate and 0.0005% of Actocol (Takeda Chemical Industry, Japan) followed by cultivation at 30°C for 24 hours. This seed culture was transferred to

- 8 -

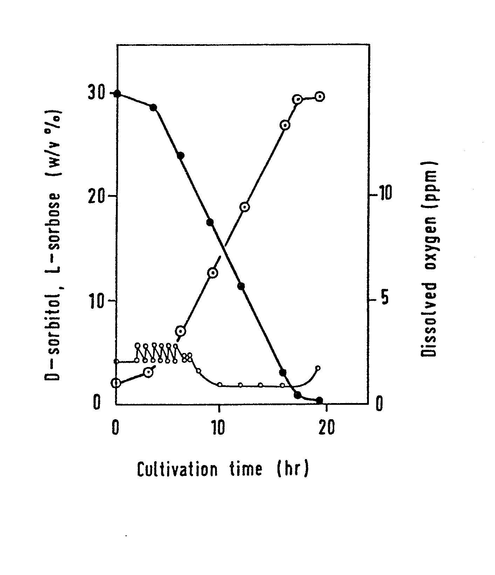

100 ℓ of a medium containing 30% of D-sorbitol, 0.039% of ammonium acetate, 0.018% of calcium carbonate, 0.003% of nicotinamide, 0.00025% of calcium pantothenate, 0.0001% of vitamin $B_2$, 0.00001% of paraaminobenzoic acid, 0.02% of potassium dihydrogen phosphate, 0.01% of magnesium sulfate, 0.00015% of ferrous sulfate and 0.00001% of manganese sulfate. Cultivation was carried out at 30°C. The rate of aeration and internal pressure were adjusted within the range of 0.1-0.6 v.v.m. and 0.1-1.5 $kg/cm^2$ gauge, respectively, to thereby maintain the dissolved oxygen concentration at a level of 2.5 ± 0.5 ppm for 9 hours after start of cultivation and, then, in the growth phase and thereafter, cultivation was carried out at an aeration rate of 0.6 v.v.m. and an internal pressure of 1.5 $kg/cm^2$ gauge: the total cultivation time was 20 hours. The relations between the dissolved oxygen concentration (DO), D-sorbitol concentration and L-sorbose concentration, on one hand, and the incubation time on the other hand as found in this cultivation are shown in Fig. 1. In the figure, -o- indicates the dissolved oxygen concentration, -●- the D-sorbitol concentration, and -⊙- the sorbose concentration. In this cultivation mode, L-sorbose was accumulated in a yield of 96.1% on the sorbitol basis.

Example 2

Gluconobacter suboxydans IFO 3257 was inoculated into 10 ℓ of a medium containing 20% of D-sorbitol, 0.2% of sodium glutamate, 0.018% of calcium carbonate, 0.003% of nicotinamide, 0.0003% of calcium pantothenate, 0.0001% of vitamin $B_2$, 0.0001% of paraaminobenzoic acid, 0.047% of potassium dihydrogen phosphate, 0.03% of yeast extract, 0.01% of magnesium sulfate, 0.00015% of ferrous sulfate, 0.00001% of manganese sulfate and 0.0005% of Actocol, followed by cultivation at 30°C for 24 hours. This seed culture was transferred to 100 ℓ of a medium containing 30%

- 9 -

of D-sorbitol, 0.029% of ammonium acetate, 0.018% of calcium carbonate, 0.003% of nicotinamide, 0.00025% of calcium pantothenate, 0.0001% of vitamin $B_2$, 0.00001% of para-aminobenzoic acid, 0.02% of potassium dihydrogen phosphate, 0.01% of magnesium sulfate, 0.00015% of ferrous sulfate and 0.00001% of manganese sulfate. Culture was carried out at 30°C for 20 hours. During this cultivation period, the aeration rate, internal pressure and stirring rate were adjusted within the ranges of 0.2-0.6 v.v.m., 0.2-1.5 $kg/cm^2$ gauge and 230-300 rpm, respectively, to thereby maintain the dissolved oxygen concentration at a level of 3.5 ± 0.5 ppm for 9 hours after start of cultivation and, then, in the growth phase and thereafter, cultivation was carried out at an aeration rate of 0.6 v.v.m., an internal pressure of 1.5 kg/$cm^2$ gauge and a stirring rate of 300 rpm. L-Sorbose was accumulated in a yield of 95.7% on the sorbitol basis.

## Example 3

Gluconobacter suboxydans IFO 12528 was inoculated into 100 ℓ of a medium containing 20% of D-sorbitol, 0.2% of sodium glutamate, 0.018% of calcium carbonate, 0.003% of nicotinamide, 0.0003% of calcium pantothenate, 0.0001% of vitamin $B_2$, 0.0001% of paraaminobenzoic acid, 0.047% of potassium dihydrogen phosphate, 0.03% of yeast extract, 0.01% of magnesium sulfate, 0.00015% of ferrous sulfate, 0.00001% of manganese sulfate and 0.0005% of Actocol, followed by cultivation at 30°C for 24 hours. This seed culture was transferred to 1,000 ℓ of a medium containing 30% of D-sorbitol, 0.039% of ammonium acetate, 0.018% of calcium carbonate, 0.003% of nicotinamide, 0.00025% of calcium pantothenate, 0.0001% of vitamin $B_2$, 0.00001% of para-aminobenzoic acid, 0.02% of potassium dihydrogen phosphate, 0.01% of magnesium sulfate, 0.00015% of ferrous sulfate and 0.00001% of manganese sulfate. Cultivation was carried out at 30°C for 20 hours. The rate of aeration and internal pressure were adjusted within the ranges of 0.1-0.5 v.v.m. and 0-1.5 kg/cm$^2$ gauge, respectively, to thereby maintain the dissolved oxygen concentration at a level of 1.5 ± 0.5 ppm for 9 hours after start of cultivation.  Then, in the growth phase and thereafter, cultivation was carried out at an aeration rate of 0.5 v.v.m. and an internal pressure of 1.5 kg/cm$^2$ gauge.  L-Sorbose was accumulated in a yield of 96.3% on the sorbitol basis.

What we claim is:

1. A method for producing L-sorbose by microbial oxidation of D-sorbitol, which comprises cultivating a competent microorganism in a medium in which the concentration of dissolved oxygen is controlled in the range of about 1 to about 4 ppm throughout the growth phase of said microorganism.

2. The method according to claim 1, wherein the variation of dissolved oxygen concentration is controlled within the range of ± about 0.3 ppm of a set value chosen from about 1 to about 4 ppm.

3. The method according to claim 1, wherein the cultivation is carried out by using a synthetic medium as main culture.

4. The method according to claim 3, wherein the synthetic medium contains ammonium acetate as the nitrogen source.

5. The method according to claim 1, wherein the microorganism is that belonging to the genus Gluconobacter.

6. The method according to claim 1, wherein the microorganism is that belonging to the species Gluconobacter suboxydans.

7. The method according to claim 1, wherein the microorganism is the species Gluconobacter oxydanse.

0199548